# EUROPEAN PATENT APPLICATION

(11) **EP 4 670 756 A1**
(43) Date of publication of application: **31.12.2025**
(21) Application number: 24185462.9
(22) Date of filing: 28.06.2024
(51) Int. Cl.: A61M 3/02

(54) **EAR CLEANING DEVICE**

(71) Applicant: Nupur Technologies, LLC, Buffalo NY14228 (US)
(72) Inventor: Priest, Joseph L., Lewiston, New York (US); Bansal, Rohan, Buffalo, New York (US)
(74) Representative: Potter Clarkson

(57) **Abstract**

A system for cleaning an ear of a user is provided. The system includes a fluid reservoir, a temperature sensor, a portable applicator, a fluid pump, and a controller. The fluid reservoir contains a fluid. The temperature sensor generates a temperature control signal corresponding to a fluid temperature of the fluid. The temperature sensor may include a thermistor. The portable applicator includes an output nozzle connected to the fluid reservoir via a fluid supply line. The portable applicator may include an output flow control trigger controlling an output flow of the fluid from the output nozzle. The fluid pump controls a flow of the fluid from the fluid reservoir to the portable applicator via the fluid supply line. The controller is operatively coupled to the fluid pump. The controller adjusts the flow of the fluid based on the temperature control signal.

## Description

### Cross-Reference to Related Applications

This application claims priority to U.S. Provisional Patent Application Serial No. 63/482,911, filed on February 2, 2023, and titled "Ear Cleaning Device," which application is herein incorporated by reference in its entirety.

### Field of the Disclosure

The present disclosure is generally directed to ear cleaning devices, and in particular to irrigation-based ear cleaning devices configured to control fluid flow according to fluid temperature.

### Background

A number of consumer ear irrigation devices are currently available. Consumer ear irrigation devices allow an individual to apply fluid to their own ear (or an ear of another individual) to remove cerumen. To remove the cerumen, the fluid typically circulates around sensitive portions of the ear, such as the ear canal and the ear drum. Fluid which is too hot may scald or burn the ear canal or ear drum. Further, fluid which is several degrees below human body temperature can create a caloric effect. As body balance is controlled by the inner ear, this caloric effect can cause vertigo or nausea. Accordingly, it is critical that the fluid being applied to the ear measures a safe and comfortable temperature.

### Summary of the Disclosure

The present disclosure relates to systems and methods for cleaning an ear of a user. The system includes a fluid reservoir, a temperature sensor, a portable applicator, a fluid pump, and a controller. The temperature sensor, which may include a thermistor, monitors fluid, such as water, stored in the fluid reservoir, and generates a temperature control signal. The fluid pump conveys a flow of the fluid from the fluid reservoir to the portable applicator via a fluid supply line. The portable applicator generates an output flow of the fluid to the ear of the user. However, if the temperature control signal indicates the temperature of the fluid breaches one or more temperature-related conditions, the controller may terminate the flow of the fluid to the portable applicator. If the flow is terminated, the fluid is prevented from reaching the ear of the user.

The temperature conditions may include an upper operable temperature threshold, a lower operable temperature threshold, and/or an operable temperature range. The flow of the fluid may be terminated if the temperature control signal indicates the temperature of the fluid is greater than the upper operable temperature threshold, less than the lower operable temperature threshold, or outside of the operable temperature range. In one embodiment, a user may program the upper operable temperature threshold, the lower operable temperature threshold, and/or the operable temperature range by entering a user input into a user interface of the system. Accordingly, the upper operable temperature threshold, the lower operable temperature threshold, and/or the operable temperature range may correspond to user comfort preferences. Similarly, the temperature conditions may also include an upper safety temperature threshold and/or a lower safety temperature threshold. The upper safety temperature threshold and/or the lower safety temperature threshold may be preprogrammed into a memory of the controller to prevent the user from programming an unsafe operable threshold, thus allowing the fluid to become too hot or too cold. The output flow of the fluid may be further controlled by an output flow control trigger arranged on the portable applicator. In another embodiment, the user interface may include a temperature indicator. The temperature indicator may reflect a range of acceptable, preprogrammed temperature conditions for ear irrigation, such as the operable temperature range. In one example, the range may be between 96 degrees Fahrenheit and 104 degrees Fahrenheit. As the user adds fluid to the fluid reservoir, the temperature indicator displays whether the fluid added is within the range, and if so, where in the range. In this way, the user can add cooler or warmer fluid to both get into the range, as well as to find a more precise personal comfort level within the range.

In some examples, the system is embodied as a consumer ear irrigation device configured to be used by individuals without medical or other specialized training. Notably, in this example, the system lacks a heating element to control the temperature of the fluid reservoir. Therefore, the temperature of the fluid within the fluid reservoir is dictated by the temperature of the fluid manually added to the reservoir. Accordingly, there is a need to monitor the temperature of the fluid to ensure the fluid is not dangerously hot (resulting in burns or scalds) or dangerously cold (resulting in vertigo, nausea, and/or vomiting). The upper and lower safety temperature thresholds are used to prevent the flow of fluid if the fluid breaches either of the upper or lower safety temperature thresholds as dangerously hot or dangerously cold.

The system may also include a video otoscope configured to capture video data corresponding to the ear of the user. The video otoscope may be stored in a receptacle integrated into a base of the system. The video otoscope wirelessly transmits the captured video data to an external user device for display to the user. In some examples, the video otoscope may be electrically coupled to the external user device via a wired connection. The receptacle may be configured to charge and/or power the video otoscope.

Generally, in one aspect, a system for cleaning an ear of a user is provided. The system includes a fluid reservoir. The fluid reservoir is configured to contain a fluid.

The system further includes a temperature sensor. The temperature sensor is configured to generate a temperature control signal. The temperature control signal corresponds to a fluid temperature of the fluid. The temperature sensor may include a thermistor.

The system further includes a portable applicator. The portable applicator includes an output nozzle. The output nozzle is connected to the fluid reservoir via a fluid supply line. The portable applicator may include an output flow control trigger. The output flow control trigger may be configured to control an output flow of the fluid from the output nozzle.

The system further includes a fluid pump. The fluid pump is configured to control a flow of the fluid from the fluid reservoir to the portable applicator via the fluid supply line.

The system further includes a controller. The controller is operatively coupled to the fluid pump. The controller is configured to adjust the flow of the fluid based on the temperature control signal.

According to an example, the controller terminates the flow of the fluid if the temperature control signal indicates that the fluid temperature is greater than an upper operable temperature threshold or less than a lower operable temperature threshold. Further to this example, the system may include a user interface. The user interface may be configured to receive the upper operable temperature threshold or the lower operable temperature threshold via a user input. The upper operable temperature threshold may be approximately 104 degrees Fahrenheit. The lower operable temperature threshold may be approximately 96 degrees Fahrenheit.

According to an example, the controller terminates the flow of the fluid if the temperature control signal indicates that the fluid temperature is outside of an operable temperature range. Further to this example, the system further includes a user interface. The user interface is configured to receive the operable temperature range via a user input. The operable temperature range is from approximately 96 degrees Fahrenheit to approximately 104 degrees Fahrenheit.

According to an example, the controller terminates the flow of the fluid if the temperature control signal indicates that the fluid temperature is greater than an upper safety temperature threshold or less than a lower safety temperature threshold. The upper safety temperature threshold may be approximately 105 degrees Fahrenheit. The lower safety temperature threshold may be approximately 95 degrees Fahrenheit.

According to an example, the system further includes a video otoscope configured to capture video data and/or still image data. The video data and/or the still image data corresponds to the ear of the user. The video otoscope may be configured to transmit the video data and/or still image data to an external user device. The external user device may include a user interface configured to display the video data and/or still image data.

According to an example, the system includes a base. The base includes an integrated receptacle configured to receive the video otoscope.

Generally, in another example, a method for cleaning an ear of a user is provided. The method includes providing a fluid reservoir containing a fluid. The method further includes generating, via a temperature sensor, a temperature control signal corresponding to a fluid temperature of the fluid. The method further includes providing a portable applicator comprising an output nozzle connected to the fluid reservoir via a fluid supply line. The method further includes controlling, via a fluid pump, a flow of the fluid from the fluid reservoir to the portable applicator via the fluid supply line. The method further includes adjusting, via a controller operatively coupled to the fluid pump, the flow of the fluid based on the temperature control signal.

According to an example, the method may further include terminating, via the controller, the flow of the fluid if the temperature control signal indicates that the fluid temperature is greater than an upper operable temperature threshold, less than a lower operable temperature threshold, or outside of an operable temperature range.

According to an example, the method may further include receiving, via a user interface, a user input corresponding to the upper operable temperature threshold, the lower operable temperature threshold, or the operable temperature range.

According to an example, the method may further include capturing, via a video otoscope, video data and/or still image data corresponding to the ear of the user. The method may further include transmitting, via the video otoscope, the video data and/or the still image data to an external user device. The method may further include displaying, via a display of the external user device, the video data and/or the still image data.

According to an example, the method may further include actuating an output flow control trigger of the portable applicator to control an output flow of the fluid from the output nozzle.

In various implementations, a processor or controller may be associated with one or more storage media (generically referred to herein as "memory," e.g., volatile and non-volatile computer memory such as RAM, PROM, EPROM, EEPROM, floppy disks, compact disks, optical disks, magnetic tape, SSD, etc.). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects as discussed herein. The terms "program" or "computer program" are used herein in a generic sense to refer to any type of computer code (e.g., software or microcode) that can be employed to program one or more processors or controllers.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### Brief Description of the Drawings

In the drawings, like reference characters generally refer to the same parts throughout the different views. Also, the drawings are not necessarily to scale, emphasis instead generally being placed upon illustrating the principles of the various embodiments.
FIG. 1 is a schematic isometric view of a system for cleaning an ear of a user according to the present disclosure.
FIG. 2 is a schematic perspective view of a portable applicator according to the present disclosure.
FIG. 3 is a block diagram of a system for cleaning an ear of a user according to the present disclosure.
FIG. 4 is a schematic front view of a base of a system for cleaning an ear of a user according to the present disclosure.
FIG. 5 is a schematic illustration of a video otoscope and an external user device according to the present disclosure.
FIG. 6 is a schematic illustration of a controller according to the present disclosure.
FIG. 7 is a flow chart illustrating the steps of a method according to the present disclosure.
FIG. 8 is a flow chart illustrating the steps of a further method according to the present disclosure.
FIG. 9 is a flow chart illustrating example optional steps of the method according to the present disclosure.

### Detailed Description of Embodiments

The present disclosure relates to systems and methods for cleaning an ear of a user. The system includes a fluid reservoir, a temperature sensor, a portable applicator, a fluid pump, and a controller. The temperature sensor, which may include a thermistor, monitors fluid, such as water, stored in the fluid reservoir, and generates a temperature control signal. The fluid pump conveys a flow of the fluid from the fluid reservoir to the portable applicator via a fluid supply line. The portable applicator generates an output flow of the fluid to the ear of the user. However, if the temperature control signal indicates the temperature of the fluid breaches one or more temperature-related conditions, the controller may terminate the flow of the fluid to the portable applicator. If the flow is terminated, the fluid is prevented from reaching the ear of the user.

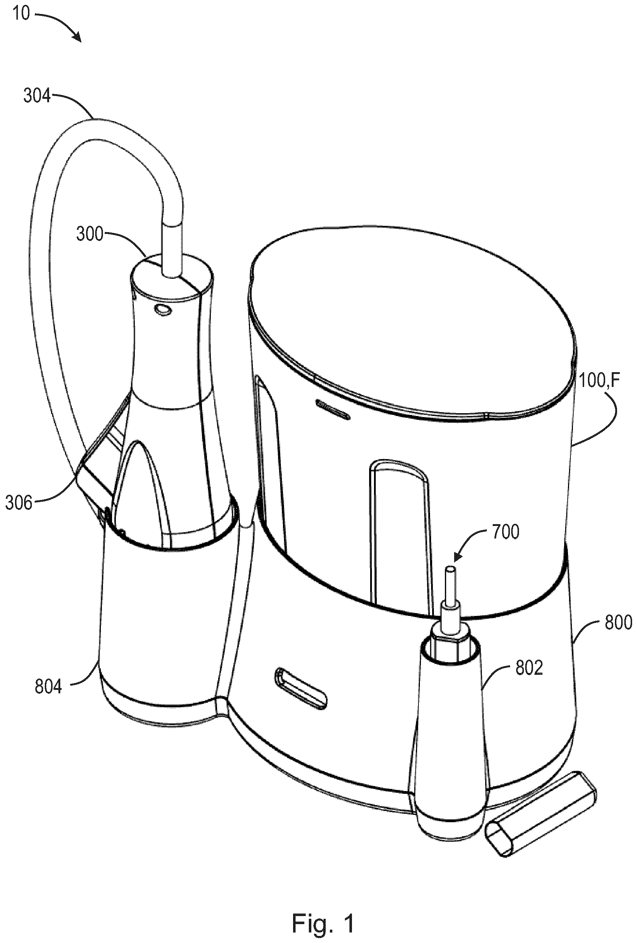

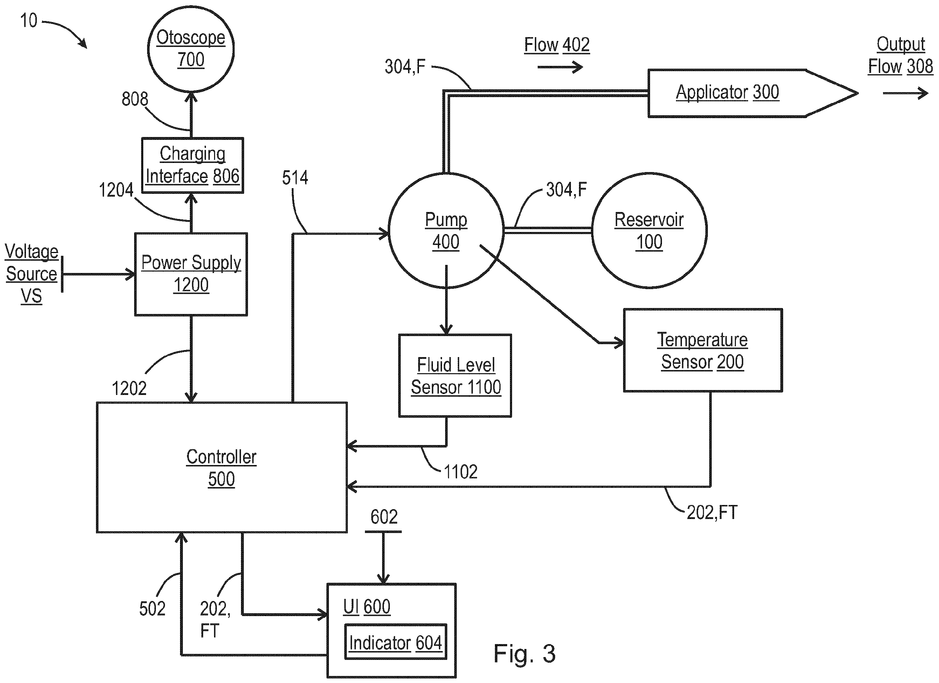

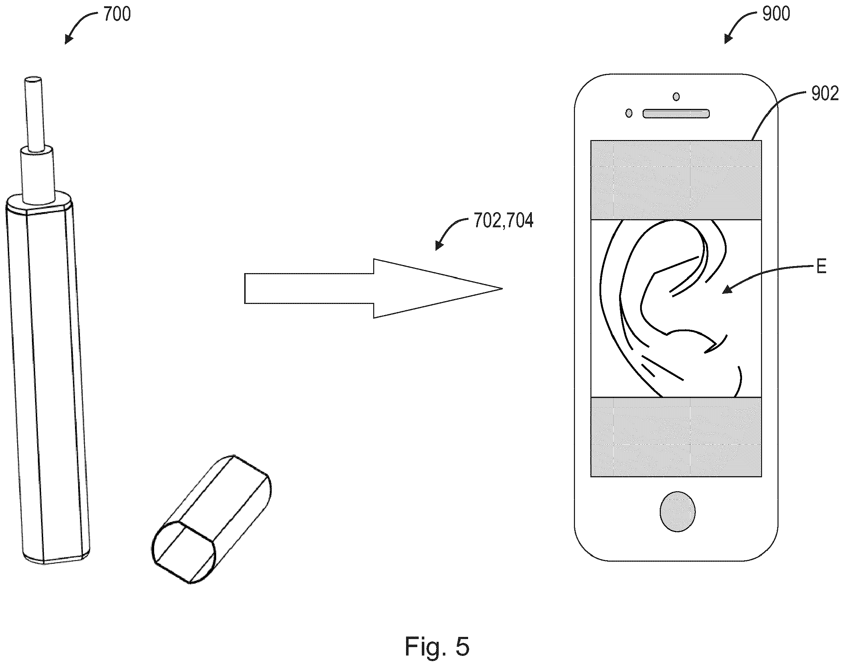

The following description should be read in view of FIGS. 1-9. FIG. 1 is a schematic illustration of a system 10 for cleaning an ear (see FIG. 5) of a user according to the present disclosure. The system 10 includes a combination of a fluid reservoir 100, a temperature sensor 200 (see FIG. 3), a portable applicator 300, a fluid pump 400 (see FIG. 3), a controller 500 (see FIG. 3), a user interface 600 (see FIG. 3), a video otoscope 700, and a base 800. Broadly, the system 10 is configured to safely propel fluid F out of the portable applicator 300 to remove cerumen (also known as earwax) from the ear of the user. The various aspects of the system 10, including the aforementioned temperature-related conditions, allow the system 10 to be safely operated by non-medical professionals. Accordingly, the system 10 shown in FIG. 1 may be considered a consumer ear irrigation device.

As shown in FIG. 1, the fluid reservoir 100 is configured to contain fluid F. The fluid F may be water, a water-based mixture, or any other liquid appropriate for circulation within the system 10 and aspects of the ear of the user. The fluid reservoir 100 may include a removable lid to facilitate refilling. As will be discussed in greater detail below, the fluid F is preferably at a fluid temperature FT (see FIG. 3) close to human body temperature, such as 98 degrees Fahrenheit. Deviations of fluid temperature FT away from human body temperature may cause discomfort during cleaning. Extreme deviations, such as fluid temperatures FT significantly higher or lower than human body temperature may pose safety issues to the user. As will be shown, the fluid reservoir 100 may contain additional components for monitoring and/or controlling the fluid F, such as the fluid pump 400, the temperature sensor 200, and a fluid level sensor 1100 (see FIG. 3). The fluid pump 400 is configured to drive fluid F from the fluid reservoir 100 to the portable applicator 300 via a fluid supply line 304. The temperature sensor 200, such as a thermistor, may monitor the fluid temperature FT of the fluid F. The fluid level sensor 1100 may monitor the level of fluid F within the fluid reservoir 100.

The fluid reservoir 100 may be arranged within the base 800. The base 800 may also include two integrated receptacles 802, 804. The first integrated receptacle 802 may be configured to receive the video otoscope 700, while the second integrated receptacle 804 may be configured to receive the portable applicator 300. In some non-limiting examples, the first integrated receptacle 802 may include a charging interface 806 configured to charge and/or power the video otoscope 700. The charging interface 806 may charge and/or power the video otoscope via any practical means. For example, the charging interface 806 may include a physical connector (such as a USB connector) to electrically connect to the video otoscope. In other examples, the charging interface 806 may be configured as a wireless charger designed to charge the video otoscope 700 via electromagnetic induction. In even further examples, the base 800 may omit second integrated receptable 804. In some examples, the controller 500 may be arranged within the base 800. As will be described in further detail with respect to FIG. 3, the controller 500 may be configured to control the fluid pump 400 according to information received from the temperature sensor 200 and/or the fluid level sensor 1100. In some examples, the video otoscope 700 may be a wireless otoscope. In other examples, the video otoscope 700 may be a wired otoscope.

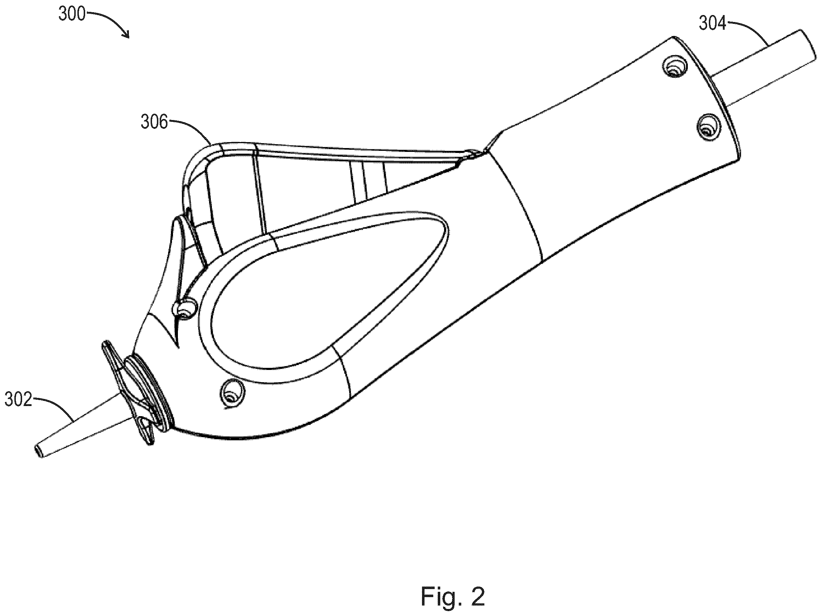

A non-limiting example of the portable applicator 300 is shown in more detail in FIG. 2. Broadly, the portable applicator 300 includes an output nozzle 302 which may be disposable, the fluid supply line 304, and an output control trigger 306. The portable applicator 300 is configured to propel fluid F out of the output nozzle 302 into the ear of the user. Accordingly, the output nozzle 302 is preferably shaped to be inserted into the ear without damaging the ear drum or other internal aspects of the ear. The portable applicator 300 receives the fluid F from the fluid reservoir 100 via the fluid supply line 304. Further, the output control trigger 306 may actuate internal components of the portable applicator 300 to control an output flow 308 (see FIG. 3) of the fluid F from the output nozzle 302. In some examples, the output control trigger 306 may function as a binary ON/OFF switch to enable or disable the output flow 308 from the output nozzle 302. In other examples, the degree of force exerted on the output control trigger 306 may be proportional of the volume of fluid F in the output flow 308.

FIG. 3 is a block diagram of a non-limiting example of the system 10 illustrated in FIG. 1. As shown in FIG. 3, the system 10 includes a fluid reservoir 100, a temperature sensor 200, a portable applicator 300, a fluid pump 400, a controller 500, a user interface 600, a video otoscope 700, a fluid level sensor 1100, and a power supply 1200. In some examples, the power supply 1200 is a low voltage, direct current (DC) isolated power supply coupled to a voltage source VS, such as an alternating current (AC) power source outputting 110 to 240 VAC. The power supply 1200 may interface with the voltage source VS via a wall outlet. The power supply 1200 generates one or more power signals 1202, 1204 to power aspects of the system 10. In the example of FIG. 3, a first power signal 1202 powers the controller 500. Further, in some non-limiting examples, a second power signal 1204 is received by a charging interface 806 configured to charge and/or power the video otoscope 700. As shown in FIG. 3, the charging interface 806 provides the video otoscope 700 with a charging signal 808. The charging signal 808 may be provided via wired connection, such as via a USB connector. Alternatively, the charging signal 808 may be provided wirelessly, such as via electromagnetic induction.

The temperature sensor 200 is configured to provide a temperature control signal 202 to the controller 500. The temperature control signal 202 is indicative of the fluid temperature FT of the fluid F within the fluid reservoir 100. The temperature sensor 200 may include a thermistor arranged within the fluid reservoir 100. The resistance of the thermistor varies according to the fluid temperature FT, thus allowing a receiving component (such as the controller 500) to determine the fluid temperature FT by processing the temperature control signal 202. In other examples, the temperature sensor 200 may incorporate other types of temperature sensing components, such as a thermocouple or a transistor-based integrated circuit (IC) sensor.

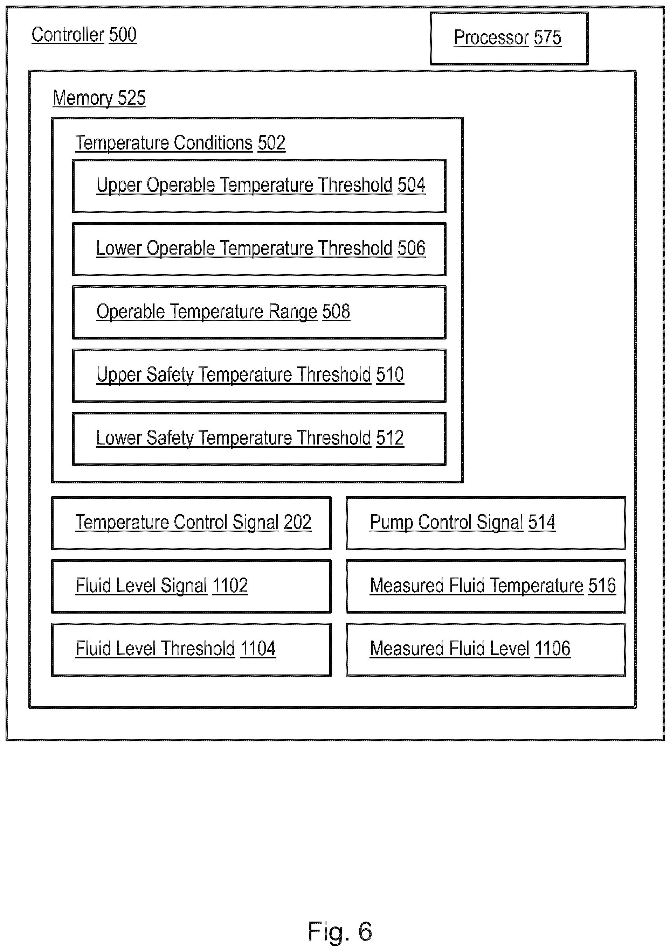

Upon receiving the temperature control signal 202, the controller 500 processes the temperature control signal 202 to determine a measured fluid temperature 516 of the fluid F. The controller 500 then compares the measured fluid temperature 516 to one or more temperature conditions 502 (see FIG. 6) stored in a memory 525 (see FIG. 6) of the controller 500. The temperature conditions 502 may include an upper operable temperature threshold 504, a lower operable temperature threshold 506, and/or an operable temperature range 508. The upper operable temperature threshold 504, the lower operable temperature threshold 506, and/or the operable temperature range 508 represent fluid temperature FT thresholds and ranges for user comfort during ear cleaning. For example, if the fluid temperature FT exceeds the upper operable temperature threshold 504, the user may experience a burning sensation as the fluid F circulates through their ear. Similarly, the temperature conditions 502 may also include an upper safety temperature threshold 510 and a lower temperature safety threshold 512. If the fluid temperature exceeds FT the upper safety temperature threshold 510, applying the fluid F may cause pain and damage to the ear of the user. Similarly, if the fluid temperature FT falls below the lower safety temperature threshold 512, the user may experience caloric stimulation, causing vertigo-like symptoms.

In some examples, one or more of the temperature conditions 502 may be programmed into memory 525 during manufacturing. For example, the upper operable temperature threshold 504, the lower operable temperature threshold 506, and/or the operable temperature range 508 could be preprogrammed based on anticipated user comfort levels. In such examples, the upper operable temperature threshold 504 could be approximately 104 degrees Fahrenheit, and the lower operable temperature threshold 506 could be approximately 96 degrees Fahrenheit. Similarly, the operable temperature range 508 could be from approximately 96 degrees Fahrenheit to approximately 104 degrees Fahrenheit.

In some examples, one or more of the temperature conditions 502 may be programmed by the user via a user interface 600. The user interface 600 could include a display screen (such as an LCD screen, a touch screen, seven segment display, etc.) and one or more input buttons. The input buttons may be physical buttons or virtual buttons displayed by the touch screen. Using the user interface 600, a user may enter a user input 602 indicative of a level of a desired temperature condition 102. For example, the user may program the upper operable temperature threshold 504 to be 99 degrees Fahrenheit, a lower operable temperature threshold 506 of 97 degrees Fahrenheit, or an operable temperature range 508 of 97.5 degrees Fahrenheit to 100 degrees Fahrenheit. The user input 602 may also indicate the user would like to reset the temperature conditions 502 to one or more previous values, such as manufacturer or default settings. In other examples, one or more physical dials or virtual dials may be incorporated into the user interface to capture user input 102.

In some examples, one or more of the temperature conditions 502 may be programmed into memory 525 during manufacturing, and are unable to be changed during use. For example, the upper safety temperature threshold 510 and the lower safety temperature threshold 512 may be stored in memory 525 as read-only to prevent a user from modifying the safety temperature thresholds 510, 512. Modifying the safety temperature thresholds 510, 512 may enable ear cleaning at dangerously hot or cold temperatures. In such examples, the upper safety temperature threshold 510 could be approximately 105 degrees Fahrenheit, while the lower safety temperature threshold 512 could be approximately 95 degrees Fahrenheit.

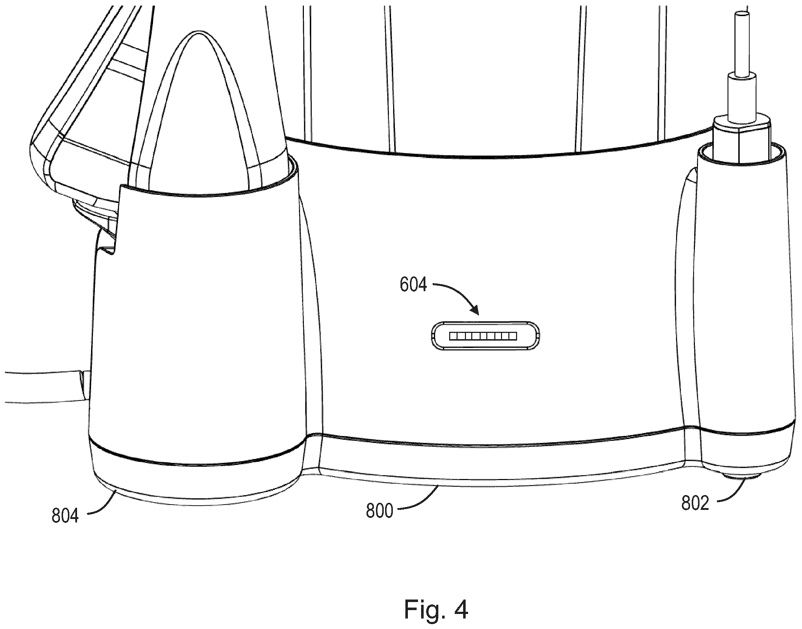

In further examples, the user interface 600 may include a temperature indicator 604 to display information regarding the fluid temperature FT of the fluid F in the fluid reservoir 100. An example of a temperature indicator 604 is shown in FIG. 4 as embedded within base 800. The indicator 604 may be embodied as one or more light sources, such as light-emitting diodes (LEDs) which illuminate according to the fluid temperature FT of the fluid F. For example, the temperature indicator 604 may include a series of LEDs from blue (representing fluid F below one or more temperature conditions 502, such as the lower operable temperature threshold 506), to green (representing fluid F within one or more temperature conditions 502, such as operable temperature range 508) to red (representing fluid F above one or more temperature conditions 502, such as the upper operable temperature threshold 504). The user interface 600 may receive the temperature control signal 202 from the controller 500, and illuminate the temperature indicator 604 accordingly. In this way, the temperature indicator 604 quickly conveys to the user whether the fluid F within the fluid reservoir 100 is at a safe and/or comfortable fluid temperature FT for ear cleaning. In some examples, the temperature indicator 604 may also display the values of the temperature conditions 502, such as via a seven-segment display. As the user adds fluid F to the fluid reservoir 100, the temperature indicator 604 displays whether the fluid F satisfies the temperature conditions 502. In this way, the user can add cooler or warmer fluid to both satisfy the temperature conditions 502, as well as to find a more precise personal comfort level within the temperature conditions 502. In further examples, the system 10 may include additional indicators, such as a fluid-level indicator showing the amount of fluid F in the fluid reservoir 100 according to the fluid-level sensor 1100.

The controller 500 generates a pump control signal 514 based on, among other factors, the temperature control signal 202. If the temperature control signal 202 indicates that the fluid temperature FT of the fluid F in the fluid reservoir 100 is at a safe and comfortable level for the user, the pump control signal 514 triggers the pump 400 to generate a flow 402 of the fluid F. The fluid F flows along the fluid supply line 304 from the fluid reservoir 100, to the fluid pump 400, to the portable applicator 300, and out of the output nozzle 302 (see FIG. 2) of the portable applicator 300 as an output flow 308. However, if the temperature control signal 202 indicates that the fluid temperature FT of the fluid F in the fluid reservoir 100 is at an unsafe or uncomfortable level for the user (such as by breaching one of the temperature thresholds 504, 506, 510, 512 or ranges 508), the pump control signal 514 disables the fluid pump 400, thus terminating the flow 402 of the fluid F. For example, if the upper operable temperature threshold 504 is 99.5 degrees Fahrenheit, and the fluid temperature FT of the fluid F in the fluid reservoir is 100 degrees Fahrenheit, the controller 500 will generate a pump control signal 514 disabling the fluid pump 400. Alternatively, in some examples, the controller 500 may disable the fluid pump 400 by ceasing to provide the pump control signal 514 to the fluid pump 400 upon the fluid temperature FT breaching the upper operable temperature threshold 504 (or any of the one or more temperature conditions 502). If the fluid temperature FT then cools to 99 degrees, the controller 500 will generate a new pump control signal 514 to trigger the fluid pump 400 to restart the flow 402 of the fluid F. Notably, in some examples, the system of FIG. 3 is embodied as a consumer ear irrigation device without a heating element for controlling fluid temperature FT. Accordingly, the combination of the temperature sensor 200, the fluid pump 400, and the controller 500, rather than a heating element, are used to ensure any fluid F provided to the portable applicator 300 is at a safe fluid temperature FT.

In further examples, the pump control signal 514 is further based on a fluid level signal 1102 generated by a fluid level sensor 1100. The fluid level sensor 1100 monitors the amount of fluid F in the fluid reservoir 100. The fluid level sensor 1100 provides the fluid level signal 1102 to the controller 500, which compares the fluid level signal 1102 to a fluid level threshold 1104 (see FIG. 6). If the fluid level signal 1102 indicates a fluid level below the fluid level threshold 1104, the controller generates a pump control signal 514 to disable the fluid pump 400 and terminate the flow 402 of fluid F in the system 10. In further examples, the fluid level sensor 1100 may mechanically interface directly with the fluid pump 400, such that the fluid level sensor 1100 mechanically disables the fluid pump 400 upon detection of the fluid level below the fluid level threshold 1104.

In further examples, the fluid reservoir 100 may be detachable. In this configuration, the fluid reservoir 100 may further include a spring plunger that is pushed up upon placement in the base 800 to enable fluid F to flow. The fluid F may flow from the detachable fluid reservoir 100 into a smaller, secondary reservoir, prior to reaching the portable applicator 300. The secondary reservoir may contain the fluid level sensor 1100 and/or the temperature sensor 200. The secondary reservoir may be arranged in front of an inlet of the fluid pump 400, such that the fluid F flows from the fluid reservoir 100, then to the secondary reservoir, then to the fluid pump 400.

FIG. 5 illustrates a video otoscope 700 and an external user device 900, embodied herein as a smartphone. As shown in FIG. 1, the base 800 of the system 10 includes an integrated receptacle 802 for a video otoscope 700. The video otoscope 700 is configured to capture video data 702 and/or still image data 704 of an ear E of a user. As shown in FIG. 5, the video otoscope 700 may then wirelessly transmit the video data 702 and/or the still image data 704 to the external user device 900. The external user device 900 receives, reconstructs, and displays the data 702, 704 on a user interface 902, such as a touch screen display. In some alternative examples, the video otoscope 700 may be communicatively coupled to the external user device 900 via a wired connection, such as a via a USB cable or other type of cable.

The video otoscope 700 is particularly useful in the example of the system 10 of FIG. 1 being used as a personal ear cleaning device. In this example, a user can use the video otoscope 700 and the external user device 900 to examine their own ear. If the video data 702 and/or still image data 704 shown by the user interface 902 of the external user device 900 shows excessive or impacted cerumen within the ear E of the user, the user may then utilize the other aspects of the system 10 to initiate ear cleaning, such as the portable applicator 300. Following cleaning, the user may again use the video otoscope 700 and the external user device 900 to confirm that their ear E has been sufficiently cleaned and excess cerumen has been removed. As shown in FIG. 5, the video otoscope 700 may be embodied as a device physically separated from the other components of the ear cleaning system 10. In some further examples, the video otoscope 700 may be integrated into the portable applicator 300 enabling the user to view their ear E not only before and after ear cleaning, as with a separate video otoscope 700, but also during ear cleaning as enabled by integrated components and/or functionality.

Notably, many presently available video otoscopes 700 are sold with scoops for a user to manually remove cerumen from their ear E. However, the use of such scoops to clean an ear E can be very dangerous, as the scoop could damage an ear drum or other internal aspects of the ear E. The system 10 provided herein overcomes this safety issue by using a portable applicator 300 (see FIG. 2) with an output nozzle 302 (see FIG. 2) incapable of reaching the ear drum. Thus, the system 10, including the video otoscope 700, allows a user to safely check their ears for cerumen, clean their ears, and check their ears for remaining cerumen without requiring the assistance of a medical professional.

FIG. 6 is a schematic diagram of a controller 500. The controller 500 includes a memory 525 and a processor 575. The memory 525 is configured to store a number of temperature conditions 502, including an upper operable temperature threshold 504, a lower operable temperature threshold 506, an operable temperature range 508, an upper safety temperature threshold 510, and a lower safety temperature threshold 512. As described above, some or all of the temperature conditions 502 may be programmed into the memory 525 during manufacturing, while some of the temperature conditions 502 may be programmed by the user via a user interface 600 (see FIG. 3). The memory 525 also stores a temperature control signal 202 received from a temperature sensor 200 (see FIG. 3) and a fluid level signal 1102 received from a fluid level sensor 1100 (see FIG. 3). The memory 525 also stores a fluid level threshold 1104 indicative of the minimum amount of fluid which may be present in the fluid reservoir 100 (see FIG. 3) to safely operate the system 10 (see FIGS. 1 and 3). The controller 500 may then process (via processor 575) the temperature control signal 202 to generate a measured fluid temperature 516 and the fluid level signal 1102 to generate a measured fluid level 1106. The processor 575 then generates a pump control signal 514 by, in part, comparing the measured fluid temperature 516 to the temperature conditions 502 and the measured fluid level 1106 to the fluid level threshold 1104. If the measured fluid temperature 516 and the measurement fluid level 1106 satisfy the aforementioned conditions, thresholds, and ranges, the pump control signal 516 is generated to trigger the fluid pump 400 (see FIG. 3) to generate a flow 402 (see FIG. 3) of fluid F (see FIG. 3) from the fluid reservoir 100 to a portable applicator 300 (see FIGS. 1-3) and to the ear E (see FIG. 5) of the user as output flow 308 (see FIG. 3). If not, the pump control signal 514 terminates the flow 402 of fluid F through the system 10. Alternatively, in some examples, the controller 500 may disable the fluid pump 400 by ceasing to provide the pump control signal 514 to the fluid pump 400.

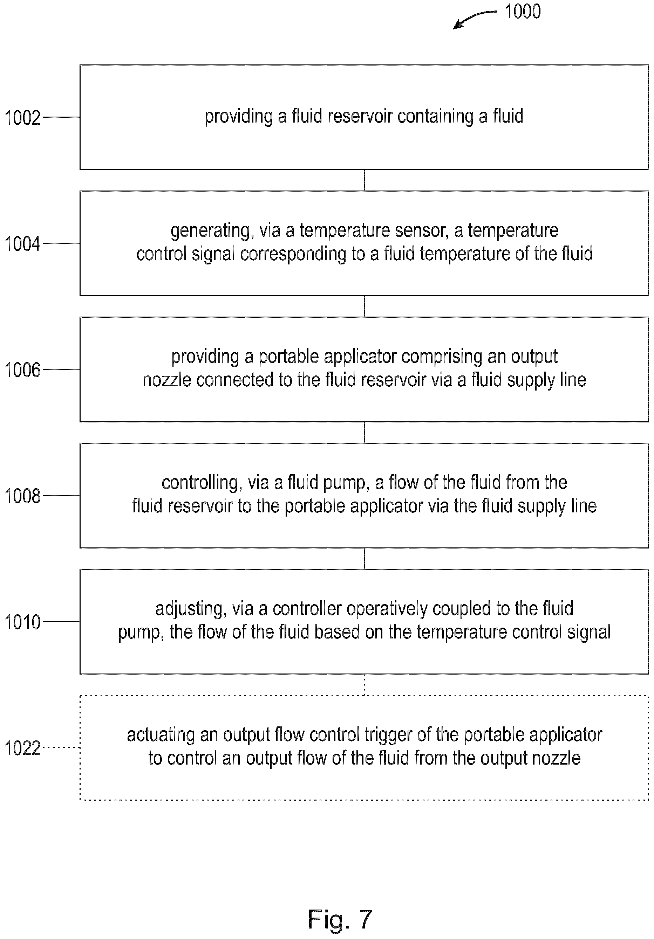

FIG. 7 is a flow chart illustrating the steps of an example method 1000 according to the present disclosure. The method 1000 includes providing 1002 a fluid reservoir containing a fluid. The method 1000 further includes generating 1004, via a temperature sensor, a temperature control signal corresponding to a fluid temperature of the fluid. The method 1000 further includes providing 1006 a portable applicator comprising an output nozzle connected to the fluid reservoir via a fluid supply line. The method 1000 further includes controlling 1008, via a fluid pump, a flow of the fluid from the fluid reservoir to the portable applicator via the fluid supply line. The method 1000 further includes adjusting 1010, via a controller operatively coupled to the fluid pump, the flow of the fluid based on the temperature control signal. The method 1000 of FIG. 7 further includes the optional step of actuating 1022 an output flow control trigger of the portable applicator to control an output flow of the fluid from the output nozzle.

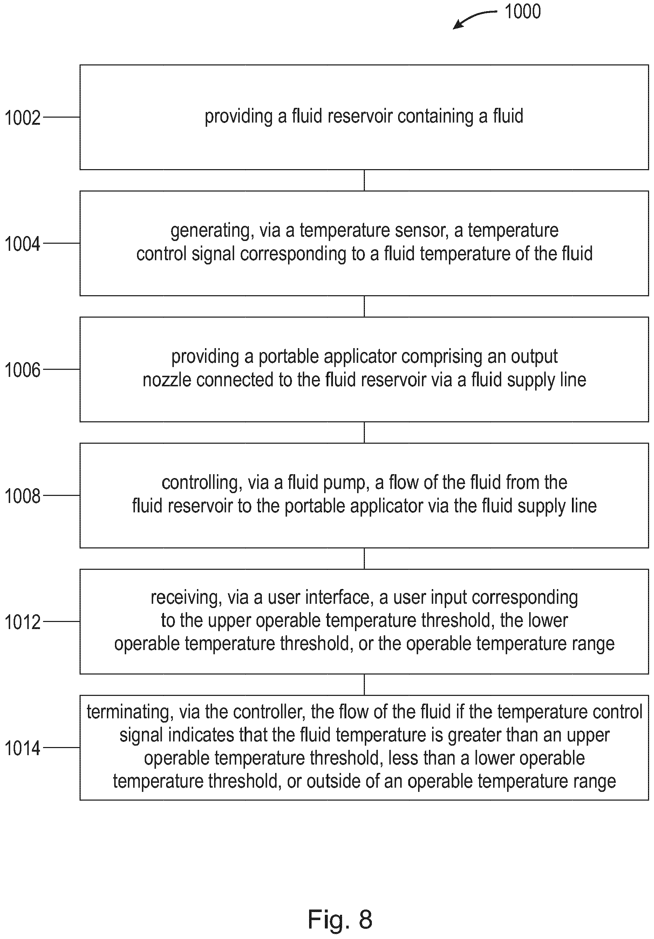

FIG. 8 is a flow chart illustrating the steps of a variation of the method 1000 for FIG. 7. Like FIG. 7, the method 1000 of FIG. 8 includes providing 1002 a fluid reservoir containing a fluid; generating 1004, via a temperature sensor, a temperature control signal corresponding to a fluid temperature of the fluid; providing 1006 a portable applicator comprising an output nozzle connected to the fluid reservoir via a fluid supply line; and controlling 1008, via a fluid pump, a flow of the fluid from the fluid reservoir to the portable applicator via the fluid supply line. The variation of FIG. 8 further includes receiving 1012, via a user interface, a user input corresponding to the upper operable temperature threshold, the lower operable temperature threshold, or the operable temperature range. The method 1000 further includes terminating 1014, via the controller, the flow of the fluid if the temperature control signal indicates that the fluid temperature is greater than an upper operable temperature threshold, less than a lower operable temperature threshold, or outside of an operable temperature range.

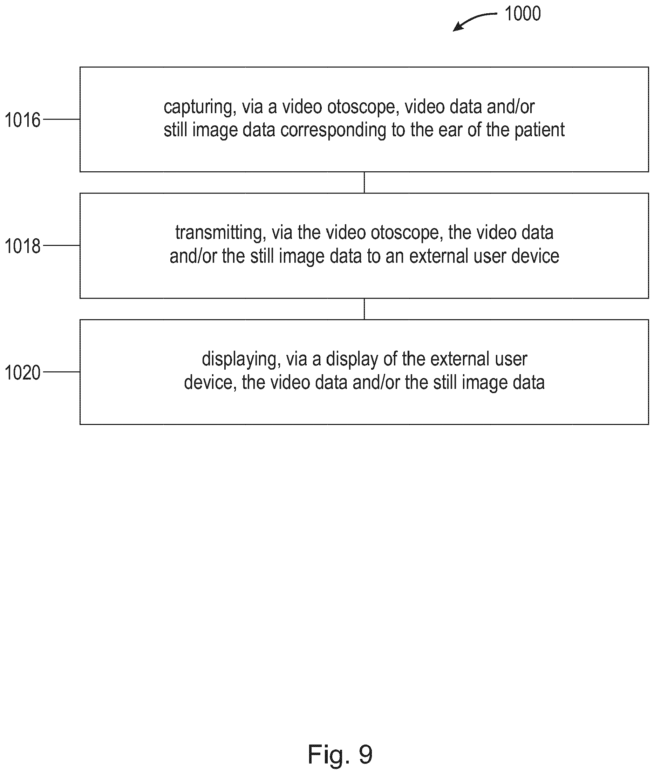

FIG. 9 is a flow chart illustrating example optional steps of the method 1000 according to the present disclosure. The optional steps include capturing 1016, via a video otoscope, video data and/or still image data corresponding to the ear of the user; transmitting 1018, via the video otoscope, the video data and/or the still image data to an external user device; displaying 1020, via a display of the external user device, the video data and/or the still image data.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

The above-described examples of the described subject matter can be implemented in any of numerous ways. For example, some aspects may be implemented using hardware, software or a combination thereof. When any aspect is implemented at least in part in software, the software code can be executed on any suitable processor or collection of processors, whether provided in a single device or computer or distributed among multiple devices/computers.

The present disclosure may be implemented as a system, a method, and/or a computer program product at any possible technical detail level of integration. The computer program product may include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some examples, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions may be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant may be entitled.

While various examples have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the examples described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific examples described herein. It is, therefore, to be understood that the foregoing examples are presented by way of example only and that, within the scope of the appended claims and equivalents thereto, examples may be practiced otherwise than as specifically described and claimed. Examples of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

## Claims

1. A system for cleaning an ear of a user, comprising:
a fluid reservoir configured to contain a fluid;
a temperature sensor configured to generate a temperature control signal corresponding to a fluid temperature of the fluid;
a portable applicator comprising an output nozzle connected to the fluid reservoir via a fluid supply line;
a fluid pump configured to control a flow of the fluid from the fluid reservoir to the portable applicator via the fluid supply line; and
a controller operatively coupled to the fluid pump and configured to adjust the flow of the fluid based on the temperature control signal.

2. The system of claim 1, wherein the controller terminates the flow of the fluid if the temperature control signal indicates that the fluid temperature is greater than an upper operable temperature threshold or less than a lower operable temperature threshold.

3. The system of claim 2, further comprising a user interface configured to receive the upper operable temperature threshold or the lower operable temperature threshold via a user input.

4. The system of claim 1, wherein the controller terminates the flow of the fluid if the temperature control signal indicates that the fluid temperature is outside of an operable temperature range.

5. The system of claim 4, further comprising a user interface configured to receive the operable temperature range via a user input.

6. The system of claim 1, wherein the controller terminates the flow of the fluid if the temperature control signal indicates that the fluid temperature is greater than an upper safety temperature threshold or less than a lower safety temperature threshold.

7. The system of claim 1, wherein the temperature sensor comprises a thermistor.

8. The system of claim 1, further comprising a video otoscope configured to capture video data and/or still image data corresponding to the ear of the user.

9. The system of claim 8, wherein the video otoscope is configured to transmit the video data and/or still image data to an external user device.

10. The system of claim 9, wherein the external user device comprises a user interface configured to display the video data and/or still image data.

11. The system of claim 8, wherein the system comprises a base having an integrated receptacle configured to receive the video otoscope.

12. The system of claim 1, wherein the portable applicator comprises an output flow control trigger configured to control an output flow of the fluid from the output nozzle.

13. A method for cleaning an ear of a user, comprising:
providing a fluid reservoir containing a fluid;
generating, via a temperature sensor, a temperature control signal corresponding to a fluid temperature of the fluid;
providing a portable applicator comprising an output nozzle connected to the fluid reservoir via a fluid supply line;
controlling, via a fluid pump, a flow of the fluid from the fluid reservoir to the portable applicator via the fluid supply line; and
adjusting, via a controller operatively coupled to the fluid pump, the flow of the fluid based on the temperature control signal.

14. The method of claim 13, further comprising:
capturing, via a video otoscope, video data and/or still image data corresponding to the ear of the user;
transmitting, via the video otoscope, the video data and/or the still image data to an external user device; and
displaying, via a display of the external user device, the video data and/or the still image data.

15. The method of claim 13, further comprising actuating an output flow control trigger of the portable applicator to control an output flow of the fluid from the output nozzle.
